# EUROPEAN PATENT APPLICATION

(11) **EP 3 940 064 A1**
(43) Date of publication of application: **19.01.2022**
(21) Application number: 20773069.8
(22) Date of filing: 16.03.2020
(51) Int. Cl.: C12N 5/0786, A61K 35/15, A61P 17/00, A61P 29/00

(54) **CELL COMPOSITION, METHOD FOR PRODUCING SAME, AND PHARMACEUTICAL COMPOSITION FOR PREVENTING OR TREATING ATOPIC DISEASE COMPRISING SAME**

(30) Priority: 15.03.2019 KR 20190029795
(71) Applicant: Therabest Co., Ltd., Seongnam-si, Gyeonggi-do 13466 (KR)
(72) Inventor: KI, Young Wook, Seoul 06570 (KR); HWANG, Do Won, Gyeonggi-do 16700 (KR); PARK, Dan Bee, Gyeonggi-do 13625 (KR); KIM, Soo Min, Gyeonggi-do 16944 (KR); LEE, Hee Kyoung, Seoul 03936 (KR)
(74) Representative: dompatent von Kreisler Selting Werner - Partnerschaft von Patent- und Rechtsanwälten mbB
(86) International application number: PCT/KR2020/003577
(87) International publication number: WO 2020/189990

(57) **Abstract**

The present invention relates to a cell composition, a method for producing the same, and a pharmaceutical composition for preventing or treating atopic disease including the same, wherein the cell composition according to the present invention has a proportion of interferon-gamma expressing cells that has increased to 60% or more among total cells, and as the proportion of cells continuously producing interferon-gamma is increased, atopic dermatitis can be significantly improved. In addition, it is expected that it will be possible to fundamentally treat immunological abnormalities of atopic dermatitis because the composition has no side effects and can be safely used for a long period of time.

## Description

### [Technical Field]

The present invention relates to a cell composition, a method for preparation of the same, and a pharmaceutical composition for preventing or treating atopic disease including the same.

### [Background Art]

Atopic dermatitis is a chronic inflammatory disease with severe itching and characteristic skin findings that occur in infants and children, often persisting through adults. The etiology of atopic dermatitis is known to be a combination of a variety of factors such as genetic background, immunological mechanism, and environmental factors, and the pathogenesis is very complex and various hypotheses exist. One of the strongest hypotheses is that Th2 cells become overactive as the homeostasis of Th1/Th2 cells is collapsed. Th2 cells over-activated by a specific allergen may secrete Th2 cytokines such as IL-4 and IL-31, and the secreted cytokines may induce IgE secretion by B cells and degranulation of mast cells, thus releasing several inflammatory substances.

However, in the treatment of atopic dermatitis until now, only a method of suppressing overall activity of immune cells using a substance such as an antiinflammatory agent, for example, corticosteroid has been used, and the above method involves a very short duration and severe side effects, thereby not being recommended as an appropriate treatment method. Further, recently developed low-molecular and antibody therapeutics have limitations in treating chronic dermatitis mediated by a complex inflammatory signaling system, such as atopic dermatitis, because they block only a receptor bound to one inflammatory substance. Further, it is not easy to avoid the risk of recurrence through other routes. Recently, there have been attempts to improve atopic dermatitis by inhibiting the activity of Th2 cells using IFN-γ protein, a type of Th1 cytokine, but IFN-γ protein has a limitation in therapeutic effects due to its extremely short half-life in the body.

Accordingly, it is very important to maximize therapeutic effects by a fundamental treatment approach through balancing the Th1/Th2 immune system in accordance with the etiology of atopic dermatitis, and it is urgent to develop a therapeutic agent for atopic dermatitis that does not cause side effects.

### [Summary of Invention]

### [Problems to be Solved by Invention]

An object of the present invention is to provide a cell composition with a proportion of 60% or more of interferon-gamma expressing cells.

In addition, another object of the present invention is to provide a method for preparation of the cell composition.

Further, another object of the present invention is to provide a pharmaceutical composition for preventing or treating atopic dermatitis, which includes the cell composition.

### [Means for Solving Problems]

To achieve the above objects, the following technical solutions are adopted in the present invention.
1. A method for production of a cell composition with a proportion of 60% or more of interferon-gamma expressing cells, including: separating and obtaining mononuclear cells ("monocytes") and autologous plasma from human peripheral blood; coating a cell culture vessel with anti-CD3 antibody; and seeding the monocytes into the cell culture vessel and culturing the same in a medium containing at least one selected from the group consisting of IL-2, IL-12 and IL-18.
2. The method according to the above 1, wherein a proportion of NKG2D-expressing cells in the composition is 60% or more.
3. The method according to the above 1, wherein one or more genes selected from the group consisting of KSP37 (Killer-specific secretory protein of 37 kDa), GNLY (Granulysin), CD74 (Cluster of Differentiation 74), ZBP1 (Z-DNA-binding protein 1), CCL5 (C-C chemokine receptor type 5) and HCST (Hematopoietic Cell Signal Transducer) is expressed five times or more compared to peripheral blood mononuclear cells (PBMCs).
4. The method according to the above 1, wherein the culture is performed in three or more stages, wherein a first stage culture is conducted by seeding the monocytes in a cell culture vessel coated with the anti-CD3 antibody and adding a medium containing IL-2, IL-12 and IL-18 thereto
   ; a second stage culture is conducted by transferring the first stage culture product to a cell culture vessel not coated with the anti-CD3 antibody and adding a medium containing IL-2; and a third stage culture is conducted by incubating the second stage culture product in a medium containing IL-2, IL-12 and IL-18.
5. The method according to the above 1, wherein a concentration of the anti-CD3 antibody is 1 to 10 µg/ml, a concentration of IL-2 is 800 to 1200 IU/mg, a concentration of IL-12 is 2 to 6 ng/ml, and a concentration of IL-18 is 20 to 60 ng/ml.
6. A cell composition as a mixture of heterologous cells, which is a culture product of human peripheral blood mononuclear cells, wherein a proportion of interferon-gamma expressing cells among total cells is 60% or more.
7. The cell composition according to the above 6, wherein the proportion of interferon-gamma expressing cells among total cells in the cell composition is 80% or more.
8. The cell composition according to the above 6, wherein a proportion of NKG2D-expressing cells among total cells in the cell composition is 60% or more.
9. The cell composition according to the above 6, wherein one or more genes selected from the group consisting of KSP37 (Killer-specific secretory protein of 37 kDa), GNLY (Granulysin), CD74 (Cluster of Differentiation 74), ZBP1 (Z-DNA-binding protein 1), CCL5 (C-C chemokine receptor type 5) and HCST (Hematopoietic Cell Signal Transducer) is expressed five times or more compared to peripheral blood mononuclear cells (PBMCs).
10. The cell composition according to the above 6, wherein a total number of cells in the composition ranges from 1 × 10⁸ to 1 × 10¹⁰ cells.
11. The cell composition according to the above 6, wherein the cell composition is obtained by culturing monocytes isolated from human peripheral blood in a medium containing one or more selected from the group consisting of anti-CD3 antibody, IL-2, IL-12 and IL-18.
12. The cell composition according to the above 11, wherein the culture is performed in three or more stages, wherein a first stage culture is conducted by seeding the monocytes in a cell culture vessel coated with the anti-CD3 antibody and adding a medium containing IL-2, IL-12 and IL-18 thereto; a second stage culture is conducted by transferring the first stage culture product to a cell culture vessel not coated with the anti-CD3 antibody and adding a medium containing IL-2; and a third stage culture is conducted by incubating the second stage culture product in a medium containing IL-2, IL-12 and IL-18.
13. The cell composition according to the above 11, wherein a concentration of the anti-CD3 antibody is 1 to 10 µg/ml, a concentration of IL-2 is 800 to 1200 IU/mg, a concentration of IL-12 is 2 to 6 ng/ml, and a concentration of IL-18 is 20 to 60 ng/ml.
14. A pharmaceutical composition for preventing or treating atopic dermatitis including the cell composition according to any one of the above 6 to 13.

### [Effects of the Invention]

The cell composition according to the present invention has a proportion of interferon-gamma expressing cells increased to 60% or more among total cells, and can significantly improve atopic dermatitis as the proportion of cells that continuously produce interferon-gamma is increased. In addition, it is expected to be able to fundamentally treat immunological abnormalities of atopic dermatitis as it can be safely used for a long period of time without side effects.

### [Brief Description of Drawings]

FIG. 1 illustrates an experimental schedule of an ovalbumin (OVA) sensitizated atopic dermatitis model.
FIGs. 2 and 3 illustrate results of analyzing cell phenotypes of the cell composition of the present invention.
FIG. 4 illustrates measurement of cumulative amounts of interferon-gamma secretion in the cell composition of the present invention according to a cell culture period.
FIG. 5 illustrates measurement of change in an amount of a specific activation factor secreted in the cell composition of the present invention according to the cell culture period.
FIGs. 6 and 7 illustrate expression levels of Ksp37, GLNY, CD74, HCST, ZBP1, and CCL5 by days after activation of the cell composition of the present invention through *in vitro* stimulation.
FIG. 8 illustrates effects of EBI on improvement of atopic dermatitis induced by ovalbumin (OVA).
FIG. 9 illustrates effects of EBI on improvement of a skin barrier with atopic dermatitis induced by ovalbumin (OVA).
FIG. 10 illustrates inhibitory effects of EBI on inflammatory response induced by ovalbumin (OVA).
FIG. 11 illustrates inhibitory effect of EBI on cytokine expression induced by ovalbumin (OVA).
FIG. 12 illustrates effects of EBI on improvement of pruritus caused by ovalbumin (OVA).
FIG. 13 illustrates skin improvement effects of the cell composition administration group of the present invention in atopic dermatitis-induced mice.
FIG. 14 illustrates a change in a body weight according to routes of administration of the cell composition.
FIGs. 15 and 16 illustrate confirmation of reduction in a transdermal thickness according to administration of the cell composition of the present invention.
FIGs. 17 and 18 illustrate effects of reducing inflammatory response according to administration of the cell composition of the present invention.
FIG. 19 illustrates confirmation of reduction in IgE contained in blood according to administration of the cell composition of the present invention.
FIG. 20 illustrates a change in atopic dermatitis-related cytokines and itching-related genes in skin tissue according to administration of the cell composition of the present invention.
FIG. 21 illustrates an amount of interferon-gamma secretion in lymph nodes according to the cell composition of the present invention.

### [Mode for Carrying out Invention]

Hereinafter, the present invention will be described in detail.

The present invention relates to a method for preparation of a cell composition.

The method for preparation of the cell composition of the present invention may include a step of separating and obtaining mononuclear cells ("monocytes") and autologous plasma, respectively, from human peripheral blood.

A method for separating monocytes from peripheral blood may use methods known in the art. In general, Ficoll method is used, wherein Ficoll is a compound obtained by polymerizing sugar and epichlorohydrin with each other, and generally uses a molecular weight of about 400,000. Ficoll is used as a material that forms a density gradient to separate cells, viruses and cell organelles, etc. because it changes into a solution ranging from low viscosity to high density when dissolved in water. Peripheral blood monocytes are lighter than red blood cells, granulocytes, and dead cells contained in the blood, but are heavier than plasma, thereby being separated.

The monocytes may be isolated and cultured from autologous blood of an individual to whom the cell composition is applied. In the case of using monocytes separated from autologous blood, unnecessary autoimmune reactions are excluded, such that atopic dermatitis can be efficiently treated without side effects such as inflammation.

The method for preparation of the cell composition of the present invention may include culturing the monocytes in a medium containing at least one selected from the group consisting of anti-CD3 antibody, IL-2, IL-12 and IL-18.

When the medium contains an anti-CD3 antibody, the anti-CD3 antibody may be coated on the medium. When the anti-CD3 antibody is coated on the medium, the medium may further include one or more selected from the group consisting of IL-2, IL-12 and IL-18.

The medium may contain anti-CD3 antibody, IL-2, IL-12 and IL-18. More specifically, the anti-CD3 antibody is coated on the medium, and IL-2, IL-12, and IL-18 may be additionally included in the cell culture medium.

The anti-CD3 antibody may be used without limitation as long as it is an antibody having a property of binding to CD3. The anti-CD3 antibody may be included in a range of 0.1 to 100 µg/ml, preferably 0.5 to 50 µg/ml, and more preferably 1 to 10 µg/ml, but it is not limited thereto.

Interleukin is a generic term for proteinaceous bioactive substances produced by immune cells such as lymphocytes, monocytes, and macrophages. The composition of the present invention may include one or more selected from the group consisting of IL-2, IL-12 and IL-18 as cytokines of interleukins. IL-2 may be included in a range of 100 to 2000 IU/ml, preferably 500 to 1500 IU/ml, and more preferably 800 to 1200 IU/ml, but it is not limited thereto. IL-12 may be included in a range of 0.5 to 10 ng/ml, preferably 1 to 8 ng/ml, and more preferably 2 to 6 ng/ml, but it is not limited thereto. IL-18 may be included in a range of 1 to 100 ng/ml, preferably 10 to 80 ng/ml, and more preferably 20 to 60 ng/ml, but it is not limited thereto. The interleukin is not limited thereto, and other interleukins known to those skilled in the art may also be used without limitation as long as they coincide with the purpose of the present invention.

The medium may further contain L-glutamine. A concentration of L-glutamine is not particularly limited, and may be in a range of, for example, 0.5 to 5 mM, and preferably 1 to 3 mM.

The medium may further contain components commonly used for culturing other monocytes. For example, glycine, L-arginine, L-asparagine, L-aspartic acid, L-cystine 2HCl, L-glutamic acid, L-histidine, L-hydroxyproline, L-isoleucine, L-leucine, L-lysine hydrochloride, L-methionine, L-phenylalanine, L-proline, L-serine, L-threonine, L-tryptophan, L-tyrosine disodium salt dihydrate, L-valine, biotin, choline chloride, D-calcium pantothenate, folic acid, niacinamide, para-aminobenzoic acid, pyridoxine hydrochloride, riboflavin, thiamine hydrochloride, vitamins B12, i-inositol, calcium nitrate, magnesium sulfate, potassium chloride, sodium bicarbonate, sodium chloride, sodium phosphate dibasic anhydrous, D-glucose, glutathione, HEPES, phenol red, etc., but it is not limited thereto.

Further, the medium may be cultured by adding an additional growth factor that supports proliferation of serum or plasma and monocytes. The type of serum or plasma to be added to the medium is not particularly limited, and commercially available products derived from various animals may be used, but those derived from humans are more preferably derived from themselves. For example, a method known to those of ordinary skill in the art may be used, such as adding a combination of cytokines, or lectins that stimulate monocyte proliferation.

Furthermore, the medium may further include an additional growth factor supporting the proliferation of serum or plasma and monocytes, and may include the serum or plasma itself. The type of serum or plasma to be added to the medium is not particularly limited, and commercially available products derived from various animals can be used, but those derived from human are preferably derived from themselves. For example, human AB serum or autologous plasma may be used.

The culture can be conducted in several stages, and for example, may be performed in two or three or more stages.

When performed in two or more steps, for example, monocytes cultured in a first stage may be transferred to a new medium in a second stage and cultured. When performed in two or more stages, the first stage medium may contain (autologous) plasma, anti-CD3 antibody, L-glutamine, IL-2, IL-12 and IL-18. Further, the second stage medium may contain (autologous) plasma, L-glutamine and IL-2. More specifically, the first stage medium may contain anti-CD3 antibody and IL-2, IL-12, IL-18, and the second stage medium may contain IL-2, but may not contain the anti-CD3 antibody, IL-12 and IL-18.

When performed in three or more stages, for example, after the second stage culture, the culture product may be transferred to a new medium and cultured. The first stage medium may contain (autologous) plasma, anti-CD3 antibody, L-glutamine, IL-2, IL-12 and IL-18, the second stage medium may contain (autologous) plasma, L-glutamine, and IL-2, and the third-stage medium may contain (autologous) plasma, L-glutamine, IL-2, IL-12 and IL-18. More specifically, the first stage medium may contain anti-CD3 antibody, IL-2, IL-12 and IL-18, the second stage medium may contain IL-2, but not contain the anti-CD3 antibody, IL-12 and IL-18, and the third stage medium may contain IL-2, IL-12, and IL-18, but not contain the anti-CD3 antibody.

Anti-CD3 antibody, L-glutamine, IL-2, IL-12 and IL-18 may be included at concentrations within the above-described range, and the above-described medium may be used.

The culture may be carried out in a general cell culture method, for example, in a CO₂ incubator. The CO₂ concentration may be, for example, 1 to 10%, specifically 3 to 7%, and the temperature may be 30 to 40 °C, specifically 35 to 38 °C, but it is not limited thereto.

The culture may be carried out until the monocytes are sufficiently activated and proliferated, and for example, for 3 to 20 days, and specifically 8 to 16 days, but it is not limited thereto.

In order to improve culture efficiency, it is preferable to add a medium in relation to an increase in the number of cells during culture. A cycle of adding the medium may be once every 1 to 10 days, and specifically 1 to 7 days, for example, to prevent deterioration of the culture medium, but it is not limited thereto.

The cell composition provided by the method of the present invention is a heterogeneous cell mixture obtained by culturing monocytes derived from peripheral blood, and may include various phenotypes of cells.

The phenotype may be, for example, CD3(+)CD56(-) cells (T cells), CD3(+)CD56(+) cells (NKT cells), CD3(-)CD56(+) cells (NK cells), etc.

The cell composition provided by the method of the present invention may include interferon-gamma expressing cells in a proportion of 60% or more.

The interferon-gamma expressing cells may include, for example, interferon-gamma-expressing cells (IPN-γ(+) cells or IFN-γ releasing cells) among the cells. In this regard, the cell composition has a very high proportion of interferon-gamma expressing cells among total cells, and therefore, may improve symptoms of atopic dermatitis when administered to a subject with atopic dermatitis.

The cell composition may have a proportion of interferon-gamma expressing cells of 60% or more, and specifically, 60% or more, 70% or more, and 80% or more. The upper limit of the proportion is not particularly limited, and may be less than 100%, and specifically may be 90% or less.

The proportion of NKG2D-expressing cells in the cell composition may be 60% or more.

The NKG2D-expressing cells may be cells to express NKG2D (NKG2D(+) or NKG2D releasing cells) among total cells. In this regard, the cell composition has a high proportion of NKG2D-expressing cells among total cells. Through a high level of NKG2D mainly expressed in Th1 cells, which play a key role in regulating immune balance for atopic efficacy, high therapeutic effects against atopic dermatitis may be achieved.

The proportion of NKG2D-expressing cells in the cell composition may be 60% or more, and specifically, 60% or more, 70% or more, and 80% or more. The upper limit of the above proportion is not particularly limited, and may be less than 100%, and specifically may be 90% or less.

The cell composition may include less than 10% of each of IL-4 and IL-13 expressing cells. Specifically, it may be less than 8%, and less than 7%, etc. The cell composition is effective in preventing and treating atopic dermatitis because the proportion of cells expressing IL-4 and IL-13 among total cells is low and the secretion of cytokines such as IL-4 and IL-13 that cause atopic dermatitis is also low.

The cell composition may have a total number of cells, for example, 1 × 10⁶ to 1 × 10¹⁰, and specifically 1 × 10⁸ to 1 × 10¹⁰.

The cell composition provided by the method of the present invention may increase the expression of genes involved in immune activity. Genes involved in the immune activity may include one or more selected from the group consisting of KSP37 (Killer-specific secretory protein of 37 kDa), GNLY (Granulysin), CD74 (Cluster of Differentiation 74), ZBP1 (Z-DNA-binding protein 1), CCL5 (C-C chemokine receptor type 5) and HCST (Hematopoietic Cell Signal Transducer), but it is not limited thereto.

The cell composition may be one in which expression of the gene is increased by 5 times or more, and 10 times or more compared to PBMC. For example, the expression of GNLY may be increased by 60 times or more.

KSP37, also known as FGFBP2 (Fibroblast Growth Factor Binding Protein 2), is a Th1/Tc1 cell-specific secretion protein. This protein is produced by a subset of NK cells, γ/δ T cells, effector CD8 T cells and Th1 cells and secreted into serum. Most of the Ksp37 expressing cells may express perforin, suggesting that Ksp37 is involved in a major process of cytotoxic lymphocyte-mediated immunity.

GNLY, also known as T-Cell Activation Protein 519, is a cytolytic and inflammatory protein present in cytotoxic cells (CTL) and cytolytic granules of NK cells along with perforin and granzymes. GNLY is a member of the saposin-like protein (SAPLIP) family, exhibits broad antimicrobial activity and potent cytotoxic action against tumor cells, activates antigen presenting cells and acts as an immune alarmin. GNLY is released when cytotoxic T cells and NK cells are adhered to infected cells, and acts as a chemoattractant for T cells, monocytes and other inflammatory cells. Further, since it stimulates the expression of various cytokines including RANTES, IL-1, IL-6, IL-10 and IFN-γ, thus to induce immune balance. Further, due to its biological function to kill target cells, symptoms of atopic dermatitis may be improved by removing damaged and deformed inflammatory cells.

CD74 acts as a cell surface receptor for a cytokine macrophage migration inhibitory factor (MIF) that initiates survival pathways and cell proliferation when bound to the encoded protein. MIF is a pro-inflammatory cytokine that inducing skin inflammation, and may be secreted from the damaged cells and release damage-related molecules such as high-mobility group protein B1. Further, MIF can increase T cell activation and invasion.

HCST (=DAP10) is an adapter molecule involved in transmission of NKG2D signals expressed in CD8+ T cells, and the binding of NKG2D and HCST may produce a protein involved in immune activity that induces phosphorylation of immunoreceptor tyrosine-based activation motif (ITAM), and induces signaling cascades of Syk (spleen tyrosine kinase) and Zap70 (zeta-chain associated protein kinase 70) so as to secrete Th1 cytokines such as interferon-gamma.

When ZBP1 may modulate modulates interferon-beta if activated, thus affecting a cytosolic pattern-recognition system and activating the immune response. Increase in activity and expression of the gene by ZBP1 is related to activation ofnecroptosis. Necroptosis means removal of virus-infected cells or inhibition of virus spreading. DAMPs (DNA, HSPs, MSU, etc.) in the cell are released outside the cell when apoptotic apoptosis occurs. At this time, some DAMPs stimulate dendritic cells (DCs), and DCs matured due to the stimulation may activate T cells. Therefore, ZBP1-involved apoptosis may remove virus-infected cells, as well as mature DCs and activate T cells, thereby inducing immune activity.

CCL5 may induce Th1 cell activity, and when acting together with interferon-gamma, can induce proliferation and activity of NK cells.

As described above, atopic dermatitis is caused due to immune imbalance by immune dysfunction and may occur due to excessive activation of Th2. The cell composition provided by the method of the present invention may inhibit Th2 proliferation and activate Th1 cells to balance the immune system, thereby improving atopic dermatitis.

Further, the present invention relates to a cell composition.

The cell composition of the present invention is a heterogeneous cell mixture obtained by culturing monocytes derived from peripheral blood, and may include various phenotypes of cells.

The phenotype may include, for example, CD3(+)CD56(-) cells (T cells), CD3(+)CD56(+) cells (NKT cells), CD3(-)CD56(+) cells (NK cells), etc.

The cell composition of the present invention is a heterogeneous cell mixture that is a culture product of human peripheral blood monocytes, wherein a proportion of interferon-gamma cells among total cells is 60% or more.

The cell composition may include interferon-gamma expressing cells in a proportion of at least 60%, and specifically, 60% or more, 70% or more, and 80% or more. The upper limit of the above proportion is not particularly limited, and may be less than 100%, and specifically may be 90% or less.

The cell composition may include NKG2D-expressing cells in a proportion of at least 60%, and specifically, 60% or more, 70% or more, and 80% or more. The upper limit of the above proportion is not particularly limited, and may be less than 100%, and specifically may be 90% or less.

The cell composition is effective in treatment of atopic dermatitis because of increased amount of secreted interferon-gamma.

The cell composition may contain less than 10% of each of IL-4 and IL-13 expressing cells. Specifically, an amount of the above cells may be less than 8% and less than 7%. The cell composition is effective in preventing and treating atopic dermatitis because the proportion of cells expressing IL-4 and IL-13 among total cells is low, and therefore secretion of IL-4 and IL-13, which are cytokines causing atopic dermatitis, is low.

The cell composition may have a total number of cells, for example, 1 × 10⁶ to 1 × 10¹⁰, specifically 1 × 10⁸ to 1 × 10¹⁰.

The cell composition may be obtained by culturing monocytes isolated from human peripheral blood in a medium containing one or more selected from the group consisting of anti-CD3 antibody, IL-2, IL-12 and IL-18.

Others such as a constitutional composition of the medium and culture methods are as described above.

Further, the present invention relates to a pharmaceutical composition for preventing or treating atopic dermatitis.

The present invention is effective in prevention or treatment of atopic dermatitis since an amount of secreted interferon-gamma, including the cell composition, is large.

Matters regarding the cell composition are as described above.

Pharmaceutically acceptable carriers included in the pharmaceutical composition of the present invention are commonly used in formulation, and may include, without limitation thereof, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, etc. The pharmaceutical composition of the present invention may further include a lubricant, a wetting agent, a sweetening agent, a flavoring agent, an emulsifying agent, a suspending agent, a preservative, and the like in addition to the above components. Pharmaceutically acceptable and desirable carriers and formulations are described in detail in Remington's Pharmaceutical Sciences (19th ed., 1995). A suitable administration dosage of the pharmaceutical composition of the present invention may vary depending on factors such as a formulation method, mode of administration, age, weight, sex, degree of pathologic symptoms, food, administration time, route of administration, excretion rate, and response sensitivity. In general, a skilled practitioner may easily determine and prescribe an effective dosage for desired treatment. Meanwhile, the dosage of the pharmaceutical composition of the present invention is not limited thereto, and may be 0.01-2000 mg/kg (body weight) per day.

The pharmaceutical composition of the present invention may be administered orally or parenterally. For parenteral administration, the composition may be administered by intravenous injection, subcutaneous injection, intramuscular injection, intraperitoneal injection, transdermal administration or the like. It is preferable that the route of administration of the pharmaceutical composition of the present invention is determined according to the type of disease to be treated. For example, since the pharmaceutical composition of the present invention is used to accelerate hair growth or prevent and treat hair loss, the composition is preferably administered in a manner of topically applying to the skin.

The pharmaceutical composition of the present invention may be prepared in a unit dosage form by a formulation process using a pharmaceutically acceptable carrier and/or excipient according to any method easily carried out by persons having common knowledge in the technical field to which the present invention pertains ("those skilled in the art"). Alternatively, it may be manufactured by placing the composition it in a multi-volume container. At this time, the formulation may be in the form of a solution, suspension, or emulsion in an oil or aqueous medium, or may be in the form of an extract, powder, granule, tablet or capsule, and may additionally include a dispersant or a stabilizer.

Hereinafter, examples will be described in detail to illustrate the present invention in detail.

### Experiment material

Experimental animals are male 6-week-old Balb/C supplied from Raon Bio (Yongin, Korea), were supplied with enough solid feed (no antibiotics) and water till the day of experiment, and then were used for experiments after acclimating for 1 week under environmental conditions at temperature 23±2 °C, humidity 55±10%, a cycle of 12 hours-12 hours (light-dark cycle). All animal testing procedures were executed with the Principles of Laboratory Animal Care of the National Institutes of Health (NIH) and the approval of the Animal Experimental Ethics Committee of Chung-Ang University.

### Example

### Experiment method

### 1. Culture of Ex-vivo Boosted Immune Cell (EBI)

The blood was kept warm at room temperature, and then centrifuged twice for 3 minutes (Ace: 4, Dec: 3) at 2000 RPM. Plasma of the supernatant was collected in a new 50 ml tube, and the blood cell layer was also collected in another new 50 ml tube. Plasma was inactivated in a water bath at 56 °Cfor 30 minutes, followed by centrifugation at 2000 RPM for 3 minutes (Ace: 4, Dec: 3). The supernatant was collected in another new 50 ml tube and used as plasma. The blood cell layer collected in the 50 ml tube was diluted by adding ALyS505N-0 medium in a 1:1 ratio. Then, 4 ml of Ficoll was put into the 15 ml tube, and 8 ml of a blood-media mix was added thereto, followed by centrifugation at 400 RCF for 30 minutes (Ace: 4, Dec: 0). After discarding the upper layer of plasma (about 3 ml), a PBMC layer between the plasma and the Ficoll layer was separated as much as possible, transferred to a 50 ml tube, and sterile physiological saline was added to reach a total volume of 50 ml, followed by centrifugation at 2000 RPM for 3 minutes (Ace: 4, Dec: 3). After removing the supernatant, the cells were dispersed using 10 ml of RBC lysis buffer, and then reacted for 3 minutes while shaking. After the reaction was completed, the total volume was adjusted to 50 ml with sterile physiological saline, and then centrifuged at 2000 RPM for 3 minutes (Ace: 4, Dec: 3). Thereafter, the supernatant was removed, cells were dispersed in 1 ml of ALyS505N-0 medium, and the cells were counted to calculate the total number of cells.

A T25 flask was coated by placing 5 µl of anti-CD3 antibody (BD, Mouse antihuman, #555329), 500 µl of 10 × HBSS, and 4.5 ml of sterile physiological saline in the T25 flask (37 °C, 4 hours). The coating solution was removed from the CD3 coated T25 flask, followed by washing the flask twice with sterile physiological saline. After seeding PBMC in an amount of 1× 10⁷ cells/5 ml into the washed T25 flask, 500 µl of (autologous) plasma, 50 µl of L-glutamine (2mM), 2.8 µl of IL-2 (1,000 IU/ml), 1.5 µl of IL-12 (3 ng/ml), 1.5 µl of IL-18 (30 ng/ml) and 4.5 ml of ALyS505N-0 medium were added, followed by incubation at 37 °C in 5% CO₂ conditions.

Flasks and bags were used for cultivation of EBI. When cultured using the flask, 500 µl of (autologous) plasma, 50 µl of L-glutamine (2mM), 2.8 µl of IL-2 (1,000 IU/ml) and 4.5 ml of ALyS505N-0 medium were added to the T25 flask prepared by the above method on day 3 after PBMC seeding. On day 4 after seeding, the mixture was transferred to a T75 flask, and 1 ml of (autologous) plasma, 100 µl of L-glutamine (2mM), 5.6 µl of IL-2 (1,000 IU/ml) and 9 ml of ALyS505N-0 medium were added thereto. On day 5 after seeding, 2 ml of (autologous) plasma, 200 µl of L-glutamine (2mM), 11.2 µl of IL-2 (1,000 IU/ml) and 18 ml of ALyS505N-0 medium were added thereto. On day 6 after seeding, the mixture was transferred to a T150 flask, and 400 µl of L-glutamine (2mM), 22.4 µl of IL-2 (1,000 IU/ml), 12 µl of IL-12 (3 ng/ml), 12 µl of IL-18 (30 ng/ml) and 40 ml of ALyS505N-0 medium were added thereto. On day 7 after seeding, 500 µl of L-glutamine (2mM), 28 µl of IL-2 (1,000 IU/ml), 15 µl of IL-12 (3 ng/ml), 15 µl of IL-18 (30 ng/ml), and 50 ml of ALyS505N-0 medium were added thereto.

On day 8 after seeding, 5 ml of IL-2 (200IU/ml) was added to the culture bag and the bag was massaged. After fixing 1/3 site of the culture bag with a clamp, about 30 ml of the medium was removed. The cells of the T150 flask were removed using a scraper and then transferred to the bag. The flask was washed using the removed medium and the cells in the medium were also transferred to the bag. Only one bag was put into one incubation chamber and flattened (37 °C, 5% CO₂). On day 10 after seeding, after bag massage, 2/3 site of the bag was fixed with a clamp while removing the clamp at 1/3 site of the bag. On day 12 after seeding, the clamps at 2/3 site of the bag were removed after bag massage. On day 14 after seeding, the bag was fixed to a clasp after back massage. A tube line for harvesting the cells was wiped with 70% EtOH, and the cells were harvested using 250 ml and 50 ml tubes, respectively, followed by centrifugation at 2000 RPM for 3 minutes (Ace: 4, Dec: 3). A portion of the supernatant was put in the 50 ml tube and the cells were collected in the 50 ml tube using a culture medium to reach a total volume of 50 ml, followed by centrifugation at 2000 RPM for 3 minutes (Ace: 4, Dec: 3). After removing the supernatant, the cell pellets were dissolved and dispersed in 1 ml of the culture medium, followed by counting the cells.

### 2. Cell phenotype measurement according to culture period

### (1) Cell activation

A sample (EBI for each culture period) was centrifuged for 5 minutes at 400 × g at room temperature. After removing the supernatant, the number of cells was adjusted to 2 × 10⁶ cells/ml, and the cells were suspended with 500 µl of the culture solution. 500 µl of the culture solution was added to a microtube, and reagents were added as follows to prepare an activation solution. 500 µl of the cell suspension was added to one well of a 24-well plate, and 500 µl of the prepared activation solution was added thereto. Reaction was performed in a CO₂ incubator under conditions of 5% CO ₂ and 37 °C for 4 hours. The cell suspension remaining in the well was transferred to a 15 ml tube, washed by adding 4 ml of 1 × PBS, and then centrifuged at 400 × g for 5 minutes. After removing the supernatant, 5 ml of FCM staining buffer was added and washed, followed by centrifugation at 400 × g for 5 minutes. After removing the supernatant, 200 µl of FCM staining buffer was added in order to suspend the cells.

### (2) Surface antigen staining

2 FCM tubes per sample (EBI per culture period) were prepared, and each tube was called an iso-tube or a sample tube. 2 µl of CD45-FITC antibody was added to each tube. 100 µl of the cell suspension of (1) was dispensed into each tube, and then mixed well with a micropipette. The solution was left to stand in the dark at room temperature for 30 minutes and dyed. After standing, 1 ml of FCM staining buffer was added to each tube, followed by centrifugation at 400 × g for 5 minutes at room temperature. After removing the supernatant, 100 µl of FCM staining buffer was added to each tube in order to suspend the pellets. After adding 100 µl of IC fixing buffer to each tube, reaction was performed for at least 20 minutes in the dark at room temperature (at this time, not exceeding 1 hour).

### (3) Intracelluar staining

1 × permeabilization buffer was prepared by diluting 10 × permeabilization buffer 10 times with distilled water. After adding 1 ml of the 1 × permeabilization buffer to each tube, centrifugation was performed at 400 × g for 5 minutes at room temperature. After removing the supernatant, 100 µl of the 1 × permeabilization buffer was added to each tube to suspend the pellets. An isotype antibody was added to the iso-tube and FCM antibody was added to the sample tube. Each tube was gently mixed (vortexing) and then left for 30 minutes in the dark at room temperature, followed by dyeing. After standing, 1 ml of the 1 × permeabilization buffer was added to each tube, followed by centrifugation at 400 × g for 5 minutes at room temperature. After removing the supernatant, 300 to 400 µl of FCM staining buffer was added to each tube to suspend the pellets. At the time of measurement, it was measured in the order of iso-tube and sample tube.

### 3. Cytokine analysis

For cytokine analysis, amounts of 10 or more cytokines were simultaneously measured using a human cytokine array (ca. ARY005B). 2 ml of the analysis buffer was treated in a prepared 4 well multi-dish, and reacted for 1 hour in a rocking shaker. 1 ml of the sample was treated with the prepared assay buffer and the maximum total volume was adjusted to 1.5 ml. The sample was treated with 15 µl of human cytokine array detection antibody cocktail, mixed, and reacted at room temperature for 1 hour. A membrane was carefully removed and rinsed with 1 ml of 1 × wash buffer. Streptoavidin-HRP was diluted in 5 ml of assay buffer and each well was treated with 2 ml of diluted streptavidin-HRP The amount of each cytokine was measured by exposing the membrane for 10 minutes with an X-ray film.

### 4. Atopic dermatitis ovalbumin (OVA) sensitized atopic model

In 7-week-old female Balb/c mice, 20 µg of ovalbumin (OVA, grade V, Sigma, St. Louis, MO, USA) and 1 mg of aluminum hydroxide (Alum, Sigma) as an immunologic adjuvant, both of which were diluted in saline, were administered intraperitoneally once per week for 2 weeks to induce atopic dermatitis sensitization. After 2 weeks from intraperitoneal administration of OVA, 100 µg of OVA was applied to the cut-haired back skin of the mouse with a 1 × 1 cm patch for sensitization for 1 week and resting for 2 weeks, which were repeated 3 times in order to induce dermatitis in a local portion. Since 2 weeks after OVA sensitization, EBI (1 × 10⁶ cells/animal) and a positive control group, that is, Cyclosporin A (CsA, 2 mg/kg) were injected for 6 weeks by tail i.v. injection.

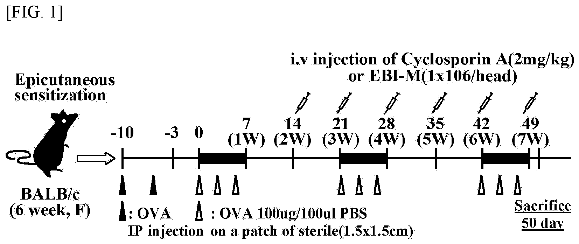

A normal (NOR) group and an OVA group were injected with physiological saline (100 µl). After completion of the experiment, blood was collected and the back skin was cut (FIG. 1).

### 5. Sensory evaluation test (evaluation of skin lesions)

As a clinical visual evaluation method commonly used in atopic dermatitis, the severity of atopic dermatitis is expressed as a sum of scores for each of the following five items. The evaluation items are erythema, pruritus & dry skin, edema & excoriation, erosion, and lichenification, etc. Each item was scored as no symptom (0 points), weak (1 point), moderate (2 points), severe (3 points) and, as a result of summing the scores of 5 items, the evaluation score of at least 0 point (with no symptoms) to the maximum of 15 points (symptoms of all items are severe) was imparted.

Skin conditions were investigated by taking pictures using a digital camera (Canon, Tokyo, Japan) immediately after the end of the experiment.

### 6. Evaluation of itching inhibition ability

A scratching behavior was measured the day before the end of the experiment in order to evaluate an ability of EBI administration to suppress itching due to atopic dermatitis. Mice were individually isolated and observed, and the behavior of hind paws that rise up to the back and down to the floor was evaluated as one time of scratch. Further, a continuous operation was considered as one time and, if scratching again after a brief interruption, the operation was included in the number of times.

### 7. Histological examination

### 1) Hematoxylin & Eosin staining

Morphological analysis was performed by Hematoxylin & Eosin staining to confirm inflammatory response induced by skin exposure to allergens. After the end of the experiment, the back tissue of the experimental animal was fixed in 10% Neutral Buffered Formalin (NBF) and, after a stepwise dehydration process from low to high concentration ethanol solution, paraffin blocks were prepared. Each tissue block was cut to a thickness of 5 µm, and the tissue sections were adhered to slides, and then each tissue slide was deparaffinized with xylene and then hydrated in alcohol. A hematoxylin reagent was added to the slide for 1 minute, followed by addition of an eosin reagent for 3 minutes, and then staining. After staining was completed, the slide was dehydrated and sealed, and then observed using an optical microscope (DM750, Leica, Wetzlar, Germany).

### 2) Toluidine blue staining

Paraffin blocks of tissues were prepared after fixing the tissues in 10% NBF to measure the number of mast cells degranulated in the ear tissues of a mouse collected through toluidine blue staining. Each tissue block was sectioned to a thickness of 5 µm and washed with distilled water after deparaffination and hydration in alcohol. Sections after washing were stained with toluidine blue (pH 0.5) for 1 hour and washed with distilled water 3-4 times. Subsequently, the number of degranulated mast cells was measured with an optical microscope (DM750, Leica, Wetzlar, Germany) after sealing through dehydration and transparent processes.

### 3) Immunohistochemical staining

The mouse skin tissue was fixed with 10% NBF and then formed into a paraffin block. Each tissue block was cut to a thickness of 5 µm, and the tissue sections were adhered to slides. After deparaffinizing each tissue slide with xylene, it was hydrated in alcohol and blocked in 5% normal serum for 1 hour. Then, the slides were treated overnight at 4°C with filaggrin, loricrin, involucrin, occludin, TRPA1 and PGP9.5 antibodies for skin barrier improvement. After washing with PBST, biotinylated rabbit anti-goat IgG (1:100, Santa Cruz Biotec) as a secondary antibody was reacted at room temperature for 24 hours, and then reacted in an avidin-biotin complex kit (Vector Lab, USA) at room temperature for 1 hour. Color was developed in 0.05 M tris-HCl buffer solution (pH 7.4) containing 0.05% 3,3'-diaminobenzidine and 0.01% HCl, followed by counter-staining with hematoxylin. Anti-TRPAl and anti-PGP9.5 were treated with FITC-conjugated secondary antibody for 1 hour. Fluorescence images were acquired using Confocal microscopy (LSM 700, ZEISS, Jena, Germany).

### 8. Measurement of total IgE in blood

1.5 mL of blood collected from a mouse was placed in a tube and centrifuged at 4 °C and 3,000 rpm for 30 minutes. After centrifugation, the supernatant was stored at -70 °C until measurement. Blood IgE was measured using an Enzyme Linked Immunosorbent Assay (ELISA). Each sample was placed in a 96-well plate treated with the primary antibody using a mouse IgE ELISA set (BD Biosciences, San Jose, CA, USA) and washed 4 times with a working solution. After washing, the sample was treated with the secondary antibody HRP-conjugated goat anti mouse IgE (1:10,000) for 1 hour, followed by color development with a color developing reagent. After completion of color development, the reaction was stopped with a stop solution, and absorbance was measured at 450 nm using ELISA.

### 9. ELISA

Quansys Q-Plex^{™} mouse cytokine array from serum and lymph isolated for analysis of cytokines (IL-1β, IL-4, IL-5, IL-6, IL-17, TNF-α and CCL2) in the serum and lymph (DonginBio, Seoul, Korea) was used to analyze the content of each cytokine. A capture antibody was diluted on the plate in a coating buffer (0.1 M sodium Carbonate, PH 9.5), sealed with a sealing tape, and adhered overnight at 4 °C. 200 µl of the assay diluent was dispensed in each well, blocked with a plate shaker for 1 hour, and 100 µl of each concentration-specific cytokine standard solution and serum were dispensed in each well and reacted again at room temperature for 2 hours. After the reaction was completed, 100 µl of the detection antibody solution was dispensed for 1 hour, and then allowed the Avidin-HRP solution to be reacted for 30 minutes. Washing of all plates between processing steps except for the last washing process was performed four times using a wash buffer (0.05% PBS-Tween 20). The final washing was performed 5 times using the wash buffer. After the end of washing, 100 µl of TMB substrate was dispensed in each well for color development and allowed to react for 20 minutes. 100 µl of the stop solution (2NH2SO4) was dispensed to complete the reaction, and absorbance at 450 nm wavelength was measured using a microplate reader and used for analysis of the content of each cytokine.

The serum was diluted by 1/2 and the supernatant was collected for lymph, and a multiplex array was conducted according to the manufacturer's instructions. 50 µl of antigen standard or sample was dispensed in each well in duplicate. After incubation for 1 hour, the plate was washed with a wash buffer, incubated with a detection mix for 1 hour, and reacted with straptavidin-HRP 1X at room temperature for 15 minutes. After washing the wells, substrates A and B were added, and an image of the plate was observed with a ChemiDoc XRS system (Bio-Rad Laboratories) and analyzed with Quansys image analysis software. To normalize cytokine data, protein concentrations were determined using a Qubit^{®} protein assay kit on a Qubit^{®} 2.0 Fluorometer (Life Technologies) and cytokine levels were expressed as pg/ml of total protein.

### 10. RNA extraction and real-time PCR

Changes in gene expression of cytokines involved in atopic dermatitis were compared and analyzed using real-time PCR. Total RNA was extracted from skin tissue and cultured cells, which were isolated using Tri-RNA Reagent (Favorgen biotech, Taiwan). Synthesis of single-stranded cDNA from a whole RNA template was performed with PrimeScript^{™} RT Master Mix (Takara, Tokyo, Japan). The generated cDNA was subjected to real-time PCR using qPCR 2x PreMIX SYBR (Enzynomics, Seoul, Korea) with CFX-96 (Bio-Rad, Hercules, CA, USA). The PCR used to amplify all genes was subjected to a denaturation process at 95 °C for 10 minutes under a condition of 40 cycles (95 °C 10 seconds, 60 °C 15 seconds, 72 °C 30 seconds). The above process was implemented a total of 40 times. Expression data was calculated as a cycle threshold (Ct) value using a ΔCt quantification method. Quantification was performed using GAPDH.

### 11. Immunoblot assay

The separated skin tissue was dissolved in PRO-PREP (iNtRON, Seongnam, Korea), and then centrifuged at 14,000 g for 20 minutes, and the supernatant was used in the experiment. A protein concentration was quantified using a BCA kit (Fisher Scientific, hampton, NH, USA). The separated supernatant (total protein amount 30 µg) was subjected to electrophoresis using 8-12% gel SDS-PAGE to isolate protein, which in turn was transferred to a PVDF membrane (Millipore, Danvers, MA, USA). The PVDF membrane containing the transferred protein was blocked in 5% skim milk for 1 hour, and the primary antibody (filaggrin, loricrin, involucrin, PAR2, TSLP, TSLPR, TRPA1, β-actin) was reacted with the membrane at 4 °C for 12 hours. After washing the reacted antibody with TBST, a secondary antibody specific to the primary antibody was reacted at room temperature for 1 hour. After washing the membrane, a color was developed with ECL solution (Millipore), and then measured using ChemiDoc^{™} XRS + System (Bio-RAD, Hercules, CA, USA).

### 12. Statistical Analysis

The experimental results were expressed as mean ± standard error mean (SEM), the significance test was performed by one way analysis of variance (ANOVA), and the post-test between groups was performed using Turkey's HDS method, and the P value of less than 0.05 was determined to be statistically significant.

### Experiment result

### 1. Cell phenotype evaluation according to culture period

In order to confirm the cell phenotype of the cell composition according to the examples, some cell samples in each culture period were collected and analyzed for phenotype, and NKG2A and NKG2D antibodies were used.

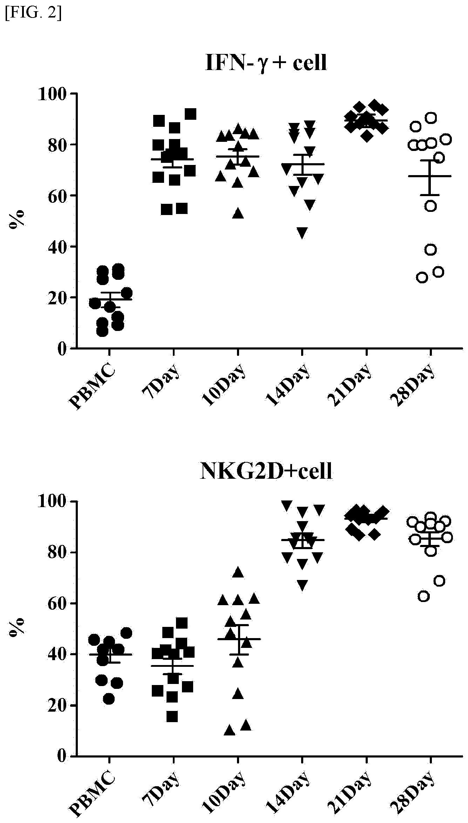

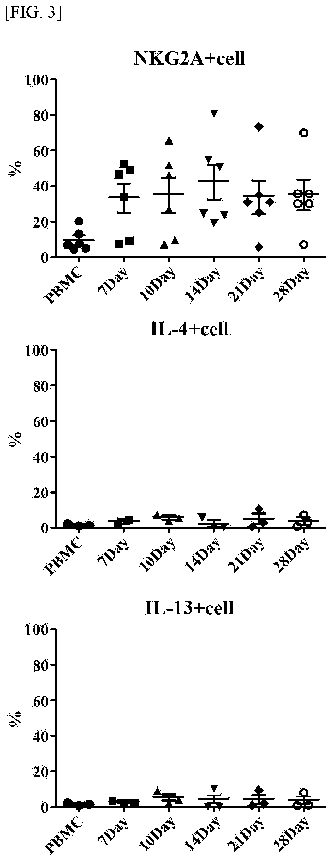

Results thereof are shown in FIGs. 2 and 3.

The proportion of interferon-gamma expressing cells (IFN-γ(+) cells, IFN-γ releasing cells) is shown in Table 1 below.

**[TABLE 1]**

| Day | 0 | 7 | 10 | 14 | 21 | 28 |
|---|---|---|---|---|---|---|
| % | 18.38 | 74.31 | 75.50 | 72.04 | 89.52 | 67.13 |

The proportion of NKG2D expressing cells (NKG2D(+) cell) is shown in Table 2 below.

**[TABLE 2]**

| Day | 0 | 7 | 10 | 14 | 21 | 28 |
|---|---|---|---|---|---|---|
| % | 39.21 | 36.30 | 45.98 | 84.19 | 93.04 | 85.17 |

The proportion of NKG2A expression cells (NKG2A(+) cell) is shown in Table 3 below

**[TABLE 3]**

| Day | 0 | 7 | 10 | 14 | 21 | 28 |
|---|---|---|---|---|---|---|
| % | 9.22 | 33.13 | 34.64 | 41.86 | 33.48 | 34.78 |

The proportion of IL-4 expressing cells (IL-4(+) cell) is shown in Table 4 below.

**[TABLE 4]**

| Day | 0 | 7 | 10 | 14 | 21 | 28 |
|---|---|---|---|---|---|---|
| % | 1.51 | 3.58 | 5.78 | 2.33 | 4.78 | 3.97 |

The proportion of IL-13 expressing cells (IL-13(+) cell) is shown in Table 5 below.

**[TABLE 5]**

| Day | 0 | 7 | 10 | 14 | 21 | 28 |
|---|---|---|---|---|---|---|
| % | 0.94 | 2.71 | 5.43 | 3.77 | 4.22 | 3.59 |

Referring to Tables 1 to 5, it can be seen that EBI has a very high proportion of cells that express interferon-gamma and NKG2D, and is effective in preventing and treating atopic dermatitis due to a low proportion of cells that express IL-4 and IL-13.

### 2. Measurement of interferon-gamma production

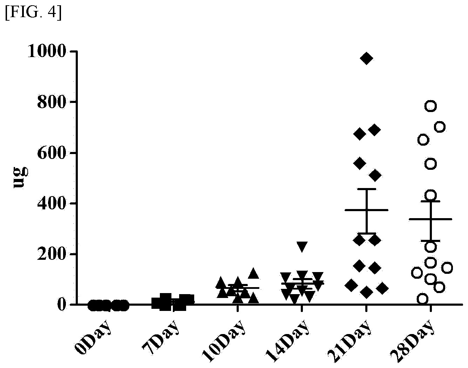

FIG. 4 and Table 6 below show measurement of cumulative amounts of extracellular interferon-gamma secretion according to the culture period. It can be seen that an amount of secretion is increased according to the culture period.

**[TABLE 6]**

| Day | 0 | 7 | 10 | 14 | 21 | 28 |
|---|---|---|---|---|---|---|
| µg | 0.00 | 15.23 | 68.64 | 86.15 | 367.97 | 331.67 |

### 3. Cytokine analysis

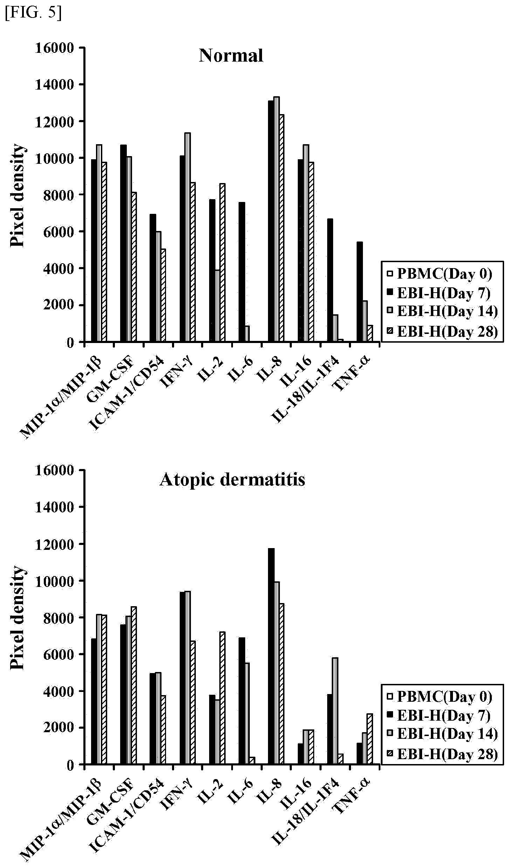

Changes in an amount of secretion of specific activation factors secreted by the culture period of the cell composition of the present invention were measured (FIG. 5).

It could be seen that levels of various immune factors involved in immune cell migration and activity were increased more than 1000 times, and IL8, IL16 and IL18 secretion was rapidly increased after 7 days. From these results, it was determined that an environment in which the cell composition of the present invention can suppress over-activated Th2 cells (for immune-suppression) generally known in atopic dermatitis through increased secretion of immune-active cells secretion factors, and may be effective in atopic dermatitis.

### 4. RNA sequencing

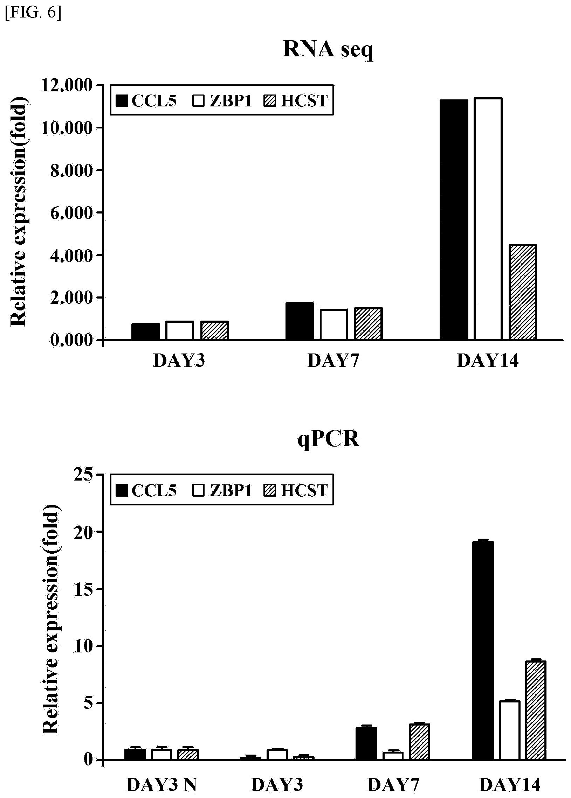

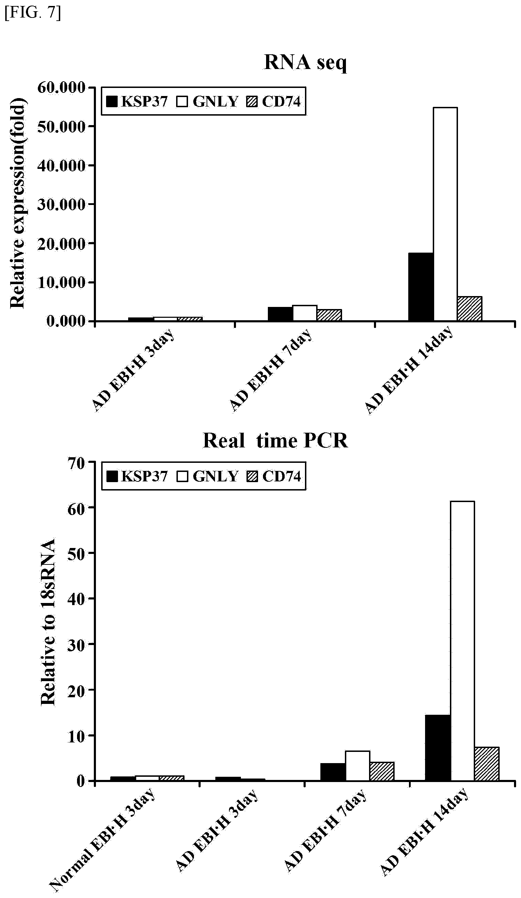

With regard to six (6) genes (Ksp37, GLNY, CD74, HCST, ZBP1, and CCL5), date-related expression levels on days 3, 7 and 14 of atopic immune cell activation were determined (FIGs. 6 and 7).

In particular, it could be found that GLNY was increased more than 60 times on day 14 in EBI. EBI could over-express the above genes to induce immune activity, thereby improving atopic dermatitis.

Tables 7 and 8 show RNA sequencing and real-time PCR results for the expression levels of Ksp37, GLNY, and CD74 by date, respectively.

Tables 9 and 10 show RNA sequencing and qPCR results for the expression levels of HCST, ZBP1, and CCL5 by date, respectively.

**[TABLE 7]**

| | KSP37 | GNLY | CD74 |
|---|---|---|---|
| Normal EBI 3 day | 1 | 1 | 1 |
| AD EBI 3 day | 0.81 | 0.25 | 0.22 |
| AD EBI 7 day | 3.93 | 6.70 | 4.21 |
| AD EBI 14 day | 14.79 | 61.61 | 7.41 |

**[TABLE 8]**

| | KSP37 | GNLY | CD74 |
|---|---|---|---|
| Normal EBI 3 day | 0.013 | 0.042 | 0.012 |
| AD EBI 3 day | 0.046 | 0.021 | 0.058 |
| AD EBI 7 day | 0.038 | 0.032 | 0.018 |
| AD EBI 14 day | 0.044 | 0.065 | 0.060 |

**[TABLE 9]**

| | DAY3 | DAY7 | DAY14 |
|---|---|---|---|
| CCL5 | 0.780 | 1.731 | 11.304 |
| ZBP1 | 0.858 | 1.417 | 11.399 |
| HCST | 0.839 | 1.498 | 4.491 |

**[TABLE 10]**

| | DAY3 Normal | DAY3 | DAY7 | DAY14 |
|---|---|---|---|---|
| CCL5 | 1 | 0.23399006397869 3 | 2.89226076232993 | 19.1779129711944 |
| ZBP1 | 1 | 0.92965471902282 | 0.80862214571731 7 | 5.2267601934814 |
| HCST | 1 | 0.30253810378102 6 | 3.15134111406133 | 8.7150560904635 |

### 5. Effect of EBI on improvement of atopic dermatitis induced by ovalbumin (OVA)

With regard to BALB/c mice, EBI (1 × 10⁶ cells/head) was injected intravenously for 6 weeks to a mouse to which atopic dermatitis-like lesions were induced by OVA skin sensitization, and an efficacy on improvement of atopic dermatitis was confirmed.

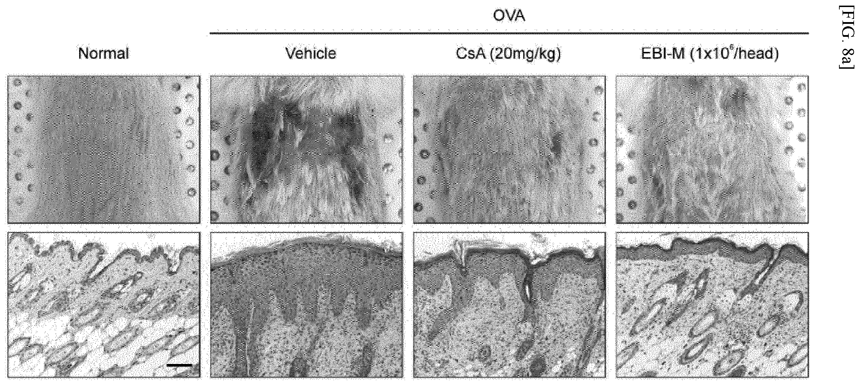

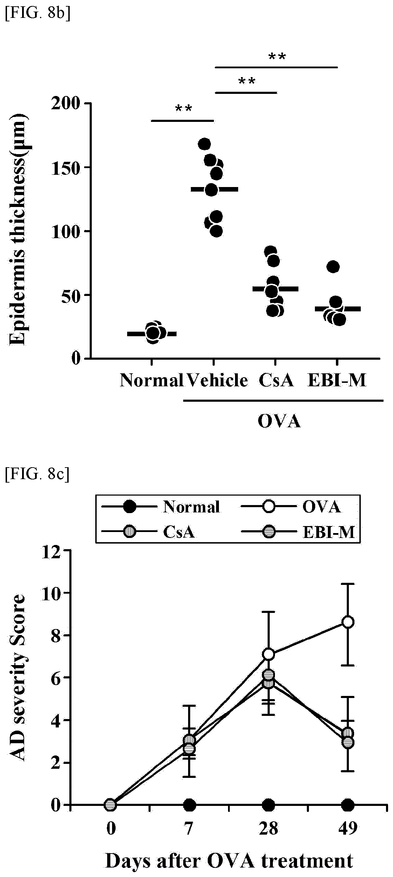

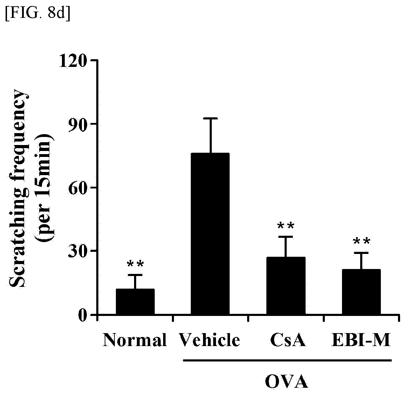

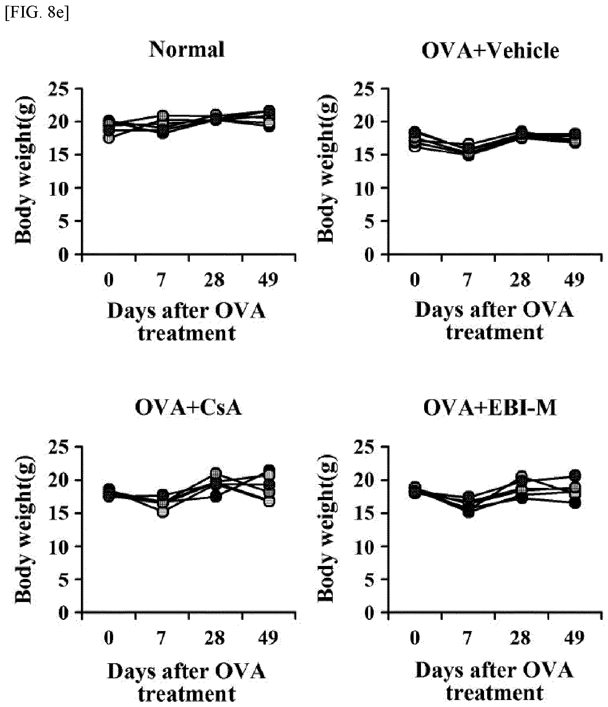

As shown in FIG. 8, it could be seen that clinical characteristics of atopic dermatitis such as skin lesion shape and skin thickness were improved in the group to which EBI was administered.

In the case of a control group induced by OVA compared to the normal mouse, dermatological symptoms (dry, erythema, soreness, swelling/hematoma, lichen) and an epidermal thickness with skin barrier collapse and inflammation were worsened, but the symptoms in the experimental group with administration of EBI showed effects of improving (FIGs. 8A to 8D). After evaluation, the skin tissue was stained with H&E and examined under a microscope to determine effects of improving skin histology. As a result, the control group induced by OVA had significantly increased epidermal thickness and skin barrier collapse compared to the normal group. The epidermis and dermis were remarkably expanded due to edema, and the removal of the epidermis and an increase in thickness could be confirmed. However, in the group to which EBI was administered, skin histological characteristics improved compared to the control group. Hyperkeratosis and parakeratosis were generally reduced, and the epidermis thickness was also decreased, leading to reduction in epidermal hyperplasia and spongiosis. Further, it could be seen that a thickness of the dermis thickened by the inflammatory reaction was also reduced, and the degenerated hair follicles were also newly formed (FIGs. 8A and 8B).

Further, in the group to which EBI was administered, the severity of atopic dermatitis (AD severity score) was improved compared to the control group (FIG. 8C), and itching was significantly reduced while the scratching behavior was relatively decreased (FIG. 8D). As a result of measuring weight change and observing effects on the animals, EBI administration did not affect the body weight of the mouse (FIG. 8E).

### 6. Effects of EBI on improvement of skin barrier of atopic dermatitis induced by ovalbumin (OVA)

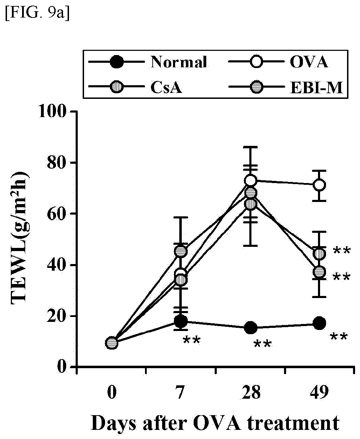

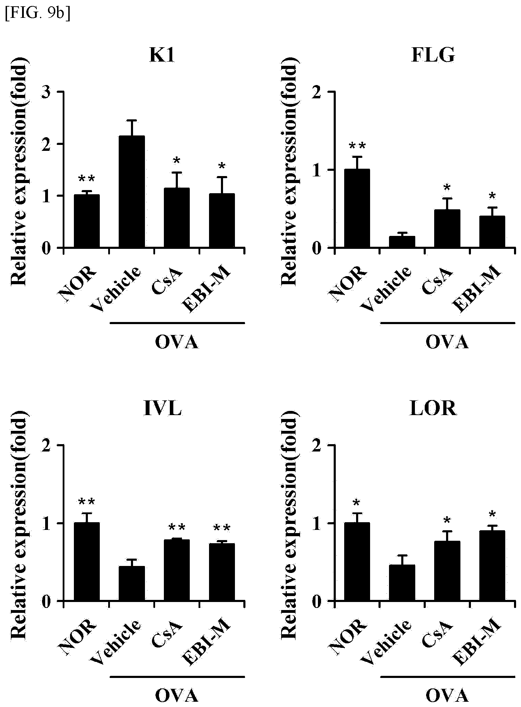

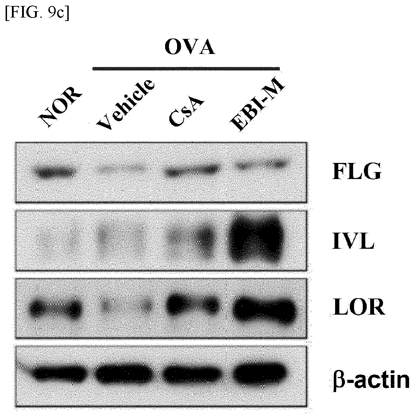

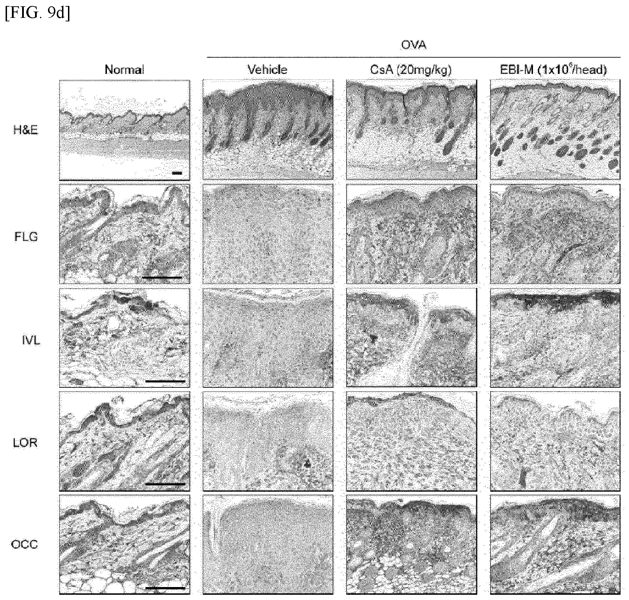

It was confirmed that an amount of epidermal moisture loss (TEWL) was significantly improved by administration of EBI (FIG. 9A). Further, as a result of measuring the expression change of keratin 1 (K1) gene in order to confirm a change in hyper-proliferation of epidermal cells, a marked decrease was observed in the EBI-administered mice (FIG. 9B). In other words, it is shown that EBI has effects of inhibiting the proliferation of epidermal cells in atopic-like lesions induced by OVA. Further, in order to measure effects of improving the skin barrier, changes in proteins to constitute the skin barrier were observed. When atopic dermatitis occurs, the expression of proteins constituting the skin barrier is reduced, thereby resulting in barrier damage, while increasing risks of water loss, penetration by allergens, and infection. Filaggrin, involucrin and loricrin are major proteins that make up the epidermal layer of the skin and are important in improving the skin barrier. Further, occludin is one of tight junction proteins and is reduced in the case of skin diseases such as atopic dermatitis. It was confirmed that OVA-induced atopic dermatitis-like lesions were improved due to administration of EBI while increasing expression of the proteins (FIGs. 9B to 9D). The administration of EBI along with the administration of CsA improved dryness symptom and was thought to effectively relieve the symptoms of atopic dermatitis by protecting the skin barrier.

### 7. Effects of EBI on inhibition of inflammatory response induced by ovalbumin (OVA)

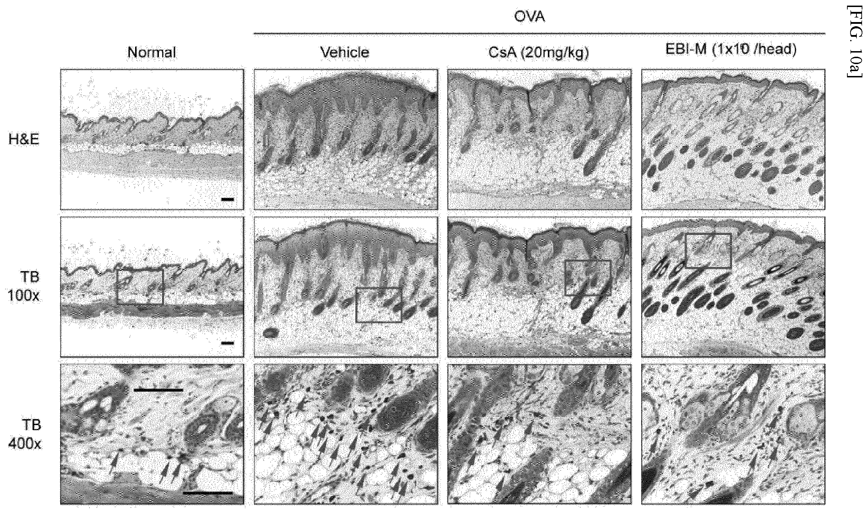

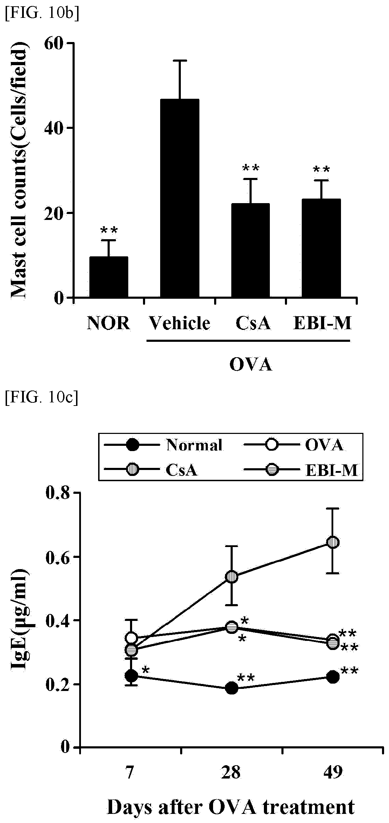

Through toluidine blue staining, phenomena related to inflammatory reactions such as mast cells could be confirmed as dark blue portions of the tissue (FIG. 10A). As a result of checking the tissue at 7 weeks (49 days) after OVA induction, in the OVA group, a lot of mast cells were infiltrated around the dermis and the thickness of the epidermis was also thicker, whereas the EBI group was observed to have reduced mast cell infiltration compared to the control group (FIGs. 10A and 10B).

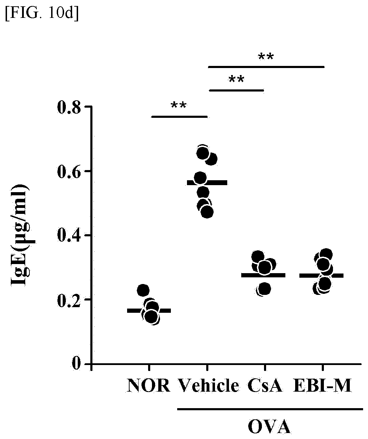

Further, in the OVA-induced atopic dermatitis model, the serum total IgE concentration (647.5 ng/mL) of the OVA sensitized group was significantly increased compared to the normal control group (NOR, 220.0 ng/mL). The increase in IgE was significantly reduced to 328.0 ng/mL and 320.5 ng/mL, respectively, by administration of CsA and EBI, thereby demonstrating significant effects (FIGs. 10C and 10D).

### 8. Effects of EBI on inhibition of cytokine expression induced by ovalbumin (OVA)

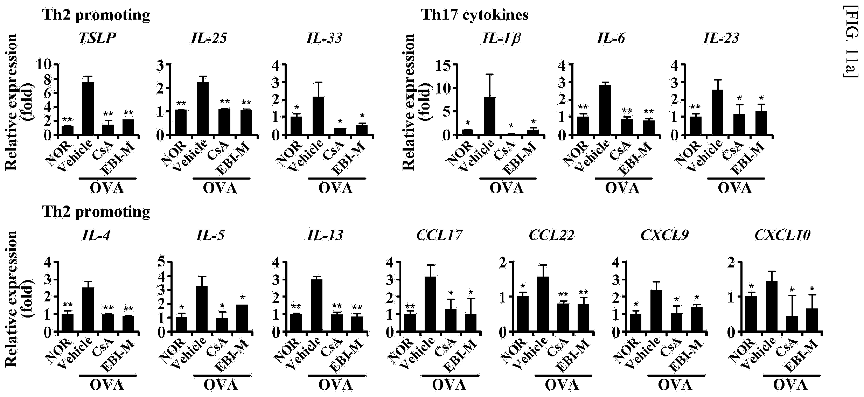

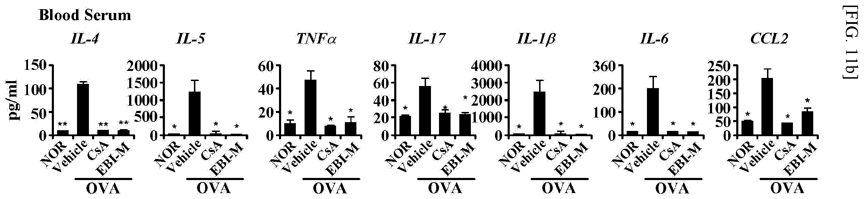

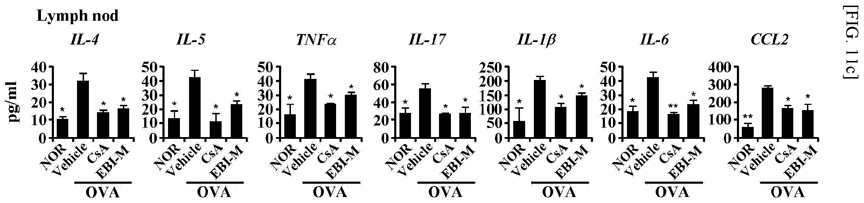

In order to confirm whether the allergic skin reaction and IgE inhibitory effects of EBI-M are related to Th2 immune response, skin tissues were isolated from OVA-sensitized mouse, and an mRNA expression level of Th2 cytokine was confirmed. As a result of the experiment, the expression of Th2 differentiation-inducing cytokines TSLP, IL-25, IL-33 and Th2 and Th17 related cytokines were significantly increased in the OVA-treated group compared to the NOR group. On the other hand, in mice administered with CsA and EBI, IL-4 was decreased by 46.8%, IL-13 was decreased by 47.3%, TSLP was decreased by 53.1% and IL-33 was decreased by 49.2%, respectively, thereby showing significant inhibition. Further, the CsA treatment groups as positive control groups also showed decreases by 33.6, 35.4, 45.8, and 57.9%, respectively, indicating that they inhibited Th2 cytokines (FIG. 11A). Further, as a result of analysis of inflammatory cytokines present in serum and lymph, IL-1β, IL-4, IL-5, TNF-α, IL-17, IL-6 and CCL2 (MCP-1) were significantly reduced by EBI administration (FIGs. 11B and 11C). Therefore, EBI suppresses the expression of Th2 cell-related cytokines, and it is believed to alleviate the symptoms of atopic dermatitis by inhibiting mast cell infiltration and IgE production, etc.

### 9. Effects of EBI on improvement of pruritus caused by ovalbumin (OVA)

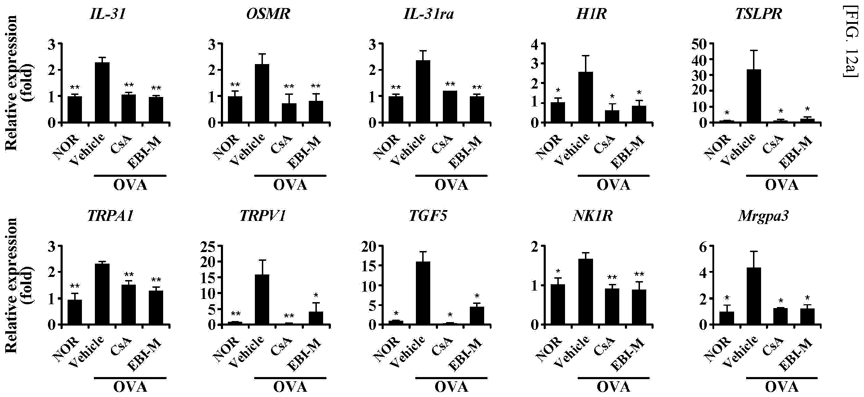

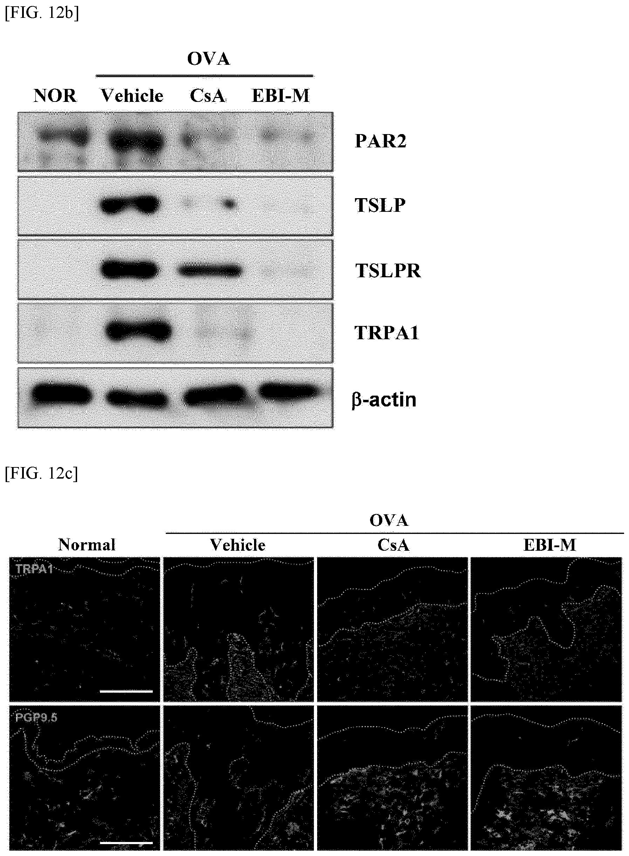

In order to observe effects of EBI on improvement of pruritus in atopic dermatitis-like lesion skin induced by OVA, the expression of various cytokines and factors known to be involved in pruritus was investigated. It can be seen that the expression of genes related to IL-31 signaling is reduced in mice administered with EBI (FIG. 12A). Further, itching stimulation is divided into two types: a histamine-dependent pathway and a histamine-independent pathway. As a result of administration of EBI, it was confirmed that the expression of genes related to itching stimulation signaling such as H1R, TRPV1, TRPA1, TGF5, NK1R, MrgpA3, TSLP, and TSLPR, etc. was significantly reduced (FIG. 12A). This result was also confirmed in terms of the protein expression level (FIG. 12B).

TSLP is also a mediator of itching associated with atopic dermatitis, and itching is induced by a histamine-independent pathway. TSLP accumulates calcium in c nerve fibers, which requires activation of TRPA1, and activation of the nerve causes itching. It was confirmed that EBI administration suppressed the expression of TRPA1 in nerve fibers in the epidermis of the skin, thereby alleviating pruritus (FIG. 12C). Further, the distribution of nerve fibers exhibiting an immune response to PGP9.5 in the skin was significantly reduced in the group administered with EBI compared to the control group, and this result was substantially consistent with a decrease in the number of neurons in the immune response of TRPA1 (FIG. 12C).

### 10. Comparison between PBMC administration group and EBI administration group

For an OVA-induced atopic dermatitis model, EBI was administered intravenously with an amount of 1 × 10⁶ cells/head once a week and total 6 times. In the case of the SC group, EBI was administered subcutaneously with an amount of 1 × 10⁶ cells/head once a week and total 6 times. Further, PBMC was administered intravenously with an amount of 1 × 10⁶ cells/head once a week and total 6 times.

### (1) Confirmation of atopic dermatitis improvement effect

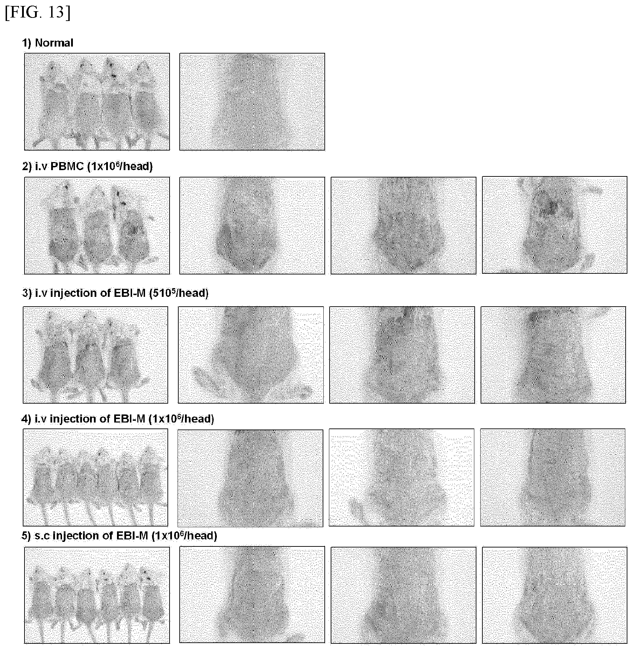

Results thereof are shown in FIG. 13.

It can be seen that skin improvement effects are excellent in the EBI-administered group with high interferon-gamma and NKG2D expression rates compared to the PBMC-administered group that did not secrete interferon-gamma.

### (2) Confirmation of body weight change according to cell composition administration

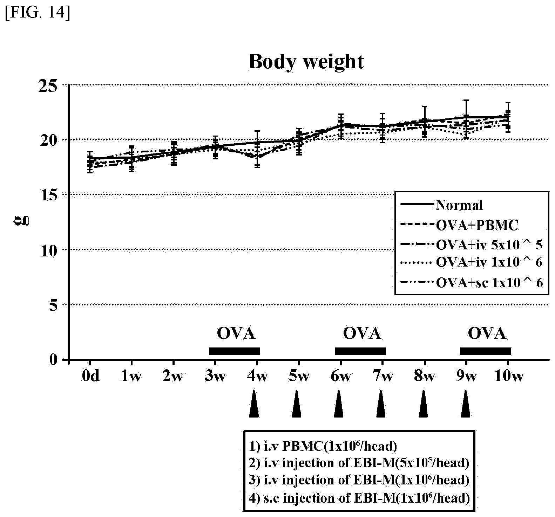

Results thereof are shown in FIG. 14. There was no noticeable change in weight in all experimental groups.

### (3) Confirmation of transdermal thickness reduction effect by administration of cell composition

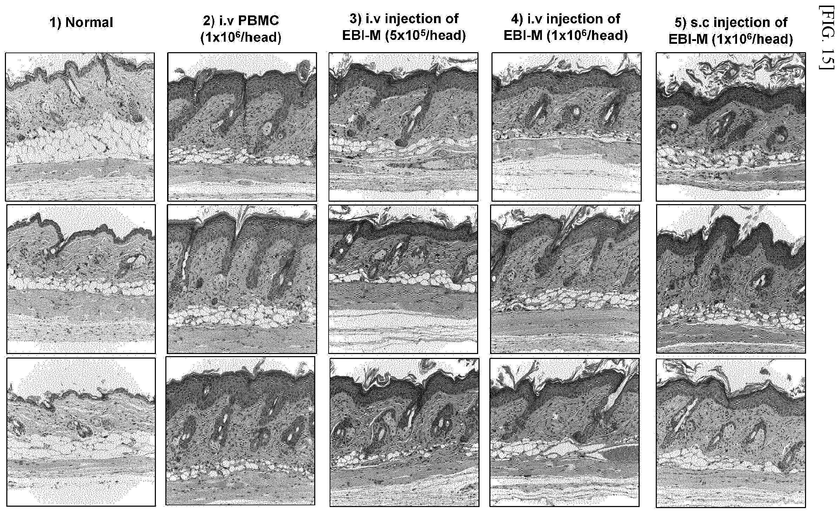

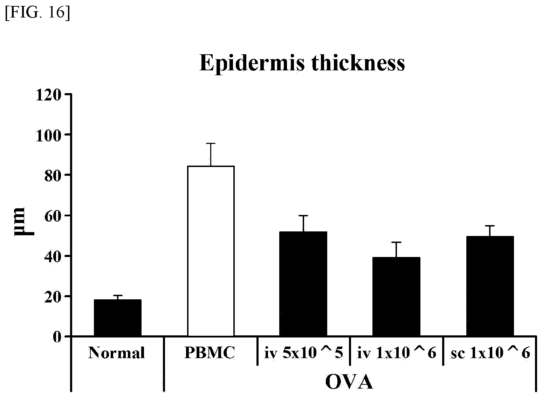

Results thereof are shown in FIGs. 15 and 16. Compared to the PBMC-administered group, the EBI-administered group with high interferon-gamma and NKG2D expression rates showed a decrease in transdermal thickness in all administration routes.

### (4) Confirmation of inflammatory response reduction effect

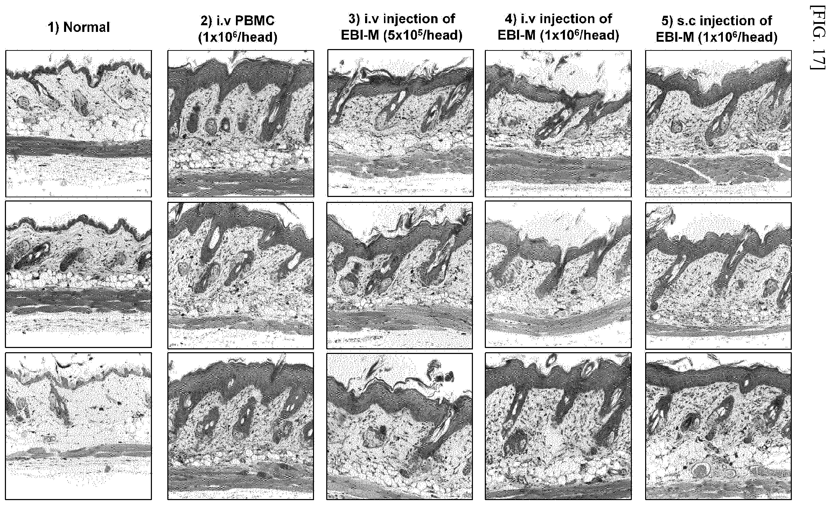

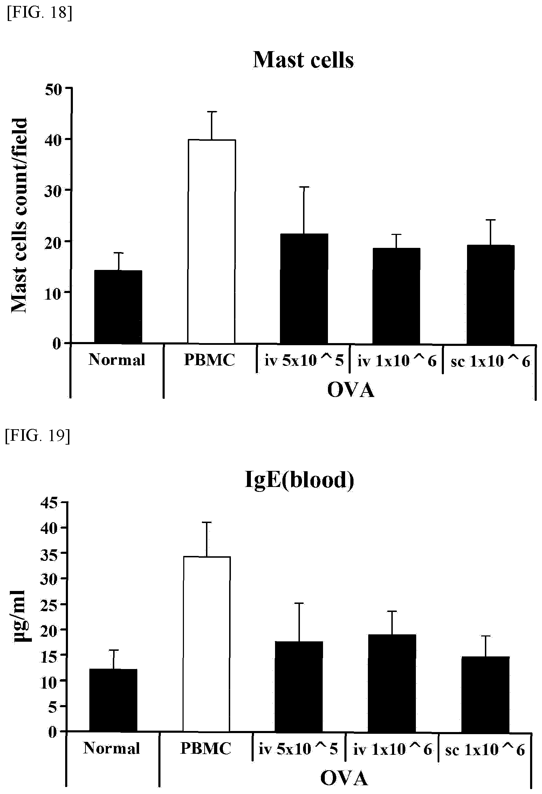

Results thereof are shown in FIGs. 17 and 18, compared to the PBMC-administered group that did not secrete interferon-gamma, the EBI-administered group with high interferon-gamma and NKG2D expression rates showed a decrease in the number of mast cells in all administration routes, thereby reducing inflammatory response.

### (5) Confirmation of IgE reduction in blood

Results thereof are shown in FIG. 19. Compared to the PBMC-administered group that did not secrete interferon-gamma, the EBI-administered group with high interferon-gamma and NKG2D expression rates showed a decrease in IgE in all administration routes.

### (6) Analysis of expression of cytokines related to atopic dermatitis and genes related to itching in skin tissue

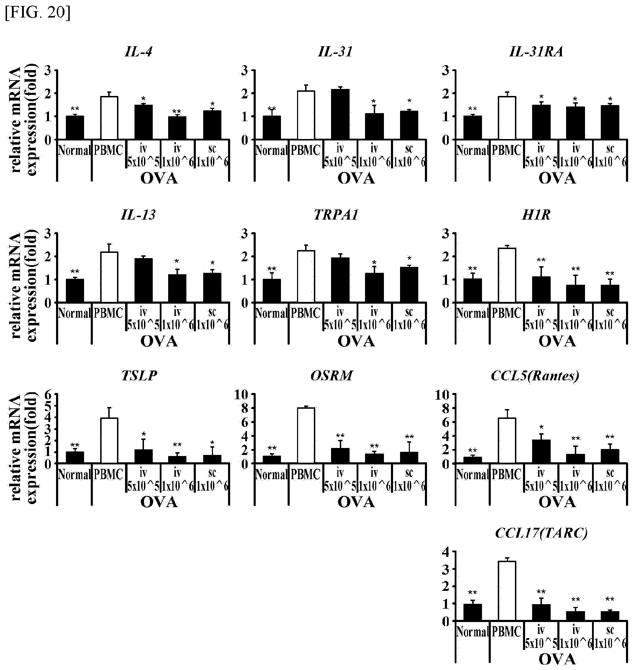

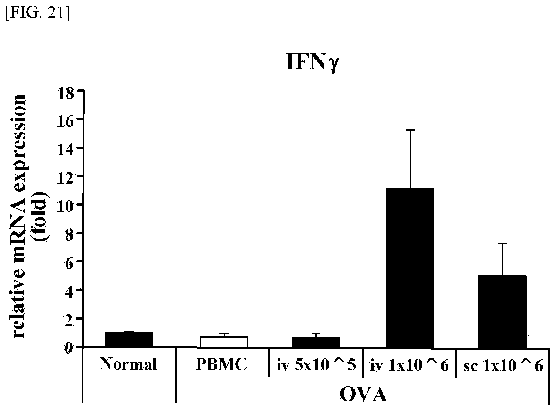

As a result, the expression of inflammatory cytokines and itching-related genes was decreased in all administration routes of the EBI-administered group with high interferon-gamma and NKG2D expression rates compared to the PBMC-administered group, and it could be seen that the EBI-administered group containing 1 × 10⁶ cells showed the highest effects (FIG. 20). In addition, interferon-gamma showed a high amount of secretion in the lymph nodes (FIG. 21)

## Claims

1. A method for production of a cell composition with a proportion of 60% or more of interferon-gamma expressing cells, comprising:
separating and obtaining mononuclear cells ("monocytes") and autologous plasma from human peripheral blood;
coating a cell culture vessel with anti-CD3 antibody; and
seeding the monocytes into the cell culture vessel and culturing the same in a medium containing at least one selected from the group consisting of IL-2, IL-12 and IL-18.

2. The method according to claim 1, wherein a proportion of NKG2D-expressing cells in the composition is 60% or more.

3. The method according to claim 1, wherein one or more genes selected from the group consisting of KSP37 (Killer-specific secretory protein of 37 kDa), GNLY (Granulysin), CD74 (Cluster of Differentiation 74), ZBP1 (Z-DNA-binding protein 1), CCL5 (C-C chemokine receptor type 5) and HCST (Hematopoietic Cell Signal Transducer) is expressed five times or more compared to peripheral blood mononuclear cells (PBMCs).

4. The method according to claim 1, wherein the culture is performed in three or more stages,
wherein a first stage culture is conducted by seeding the monocytes in a cell culture vessel coated with the anti-CD3 antibody and adding a medium containing IL-2, IL-12 and IL-18 thereto;
a second stage culture is conducted by transferring the first stage culture product to a cell culture vessel not coated with the anti-CD3 antibody and adding a medium containing IL-2; and
a third stage culture is conducted by incubating the second stage culture product in a medium containing IL-2, IL-12 and IL-18.

5. The method according to claim 1, wherein a concentration of the anti-CD3 antibody is 1 to 10 µg/ml, a concentration of IL-2 is 800 to 1200 IU/mg, a concentration of IL-12 is 2 to 6 ng/ml, and a concentration of IL-18 is 20 to 60 ng/ml.

6. A cell composition as a mixture of heterologous cells, which is a culture product of human peripheral blood mononuclear cells, wherein a proportion of interferon-gamma expressing cells among total cells is 60% or more.

7. The cell composition according to claim 6, wherein the proportion of interferon-gamma expressing cells among total cells in the cell composition is 80% or more.

8. The cell composition according to claim 6, wherein a proportion of NKG2D-expressing cells among total cells in the cell composition is 60% or more.

9. The cell composition according to claim 6, wherein one or more genes selected from the group consisting of KSP37 (Killer-specific secretory protein of 37 kDa), GNLY (Granulysin), CD74 (Cluster of Differentiation 74), ZBP1 (Z-DNA-binding protein 1), CCL5 (C-C chemokine receptor type 5) and HCST (Hematopoietic Cell Signal Transducer) is expressed five times or more compared to peripheral blood mononuclear cells (PBMCs).

10. The cell composition according to claim 6, wherein a total number of cells in the composition ranges from 1 × 10⁸ to 1 × 10¹⁰ cells.

11. The cell composition according to claim 6, wherein the cell composition is obtained by culturing monoytes isolated from human peripheral blood in a medium containing one or more selected from the group consisting of anti-CD3 antibody, IL-2, IL-12 and IL-18.

12. The cell composition according to claim 11, wherein the culture is performed in three or more stages,
wherein a first stage culture is conducted by seeding the monocytes in a cell culture vessel coated with the anti-CD3 antibody and adding a medium containing IL-2, IL-12 and IL-18 thereto;
a second stage culture is conducted by transferring the first stage culture product to a cell culture vessel not coated with the anti-CD3 antibody and adding a medium containing IL-2; and
a third stage culture is conducted by incubating the second stage culture product in a medium containing IL-2, IL-12 and IL-18.

13. The cell composition according to claim 11, wherein a concentration of the anti-CD3 antibody is 1 to 10 µg/ml, a concentration of IL-2 is 800 to 1200 IU/mg, a concentration of IL-12 is 2 to 6 ng/ml, and a concentration of IL-18 is 20 to 60 ng/ml.

14. A pharmaceutical composition for preventing or treating atopic dermatitis comprising the cell composition according to any one of claims 6 to 13.
